# EUROPEAN PATENT SPECIFICATION

(11) **EP 1 534 206 B1**
(45) Date of publication and mention of the grant of the patent: **15.08.2007**
(21) Application number: 03761039.1
(22) Date of filing: 05.06.2003
(51) Int. Cl.: A61F 13/20

(54) **COMPRESSED ABSORBENT TAMPON**
KOMPRIMIERTER SAUGFûHIGER TAMPON
TAMPON ABSORBANT COMPRIME

(30) Priority: 25.06.2002 US 179430
(43) Date of publication of application: 01.06.2005
(73) Proprietor: McNEIL-PPC, Inc., Skillman, New Jersey 08558 (US)
(72) Inventor: JONES, Archie, L., Somerset, NJ 08873 (US); LOUIE, Lai-Hing, Kendall Park, NJ 08824 (US); NGUYEN, Hein, East Windsor, NJ 08520 (US); ROLLER, Judith, E., North Brunswick, NJ 08902 (US); SAILER, Edwin, H., Ringoes, NJ 08551 (US)
(74) Representative: Metten, Karl-Heinz
(86) International application number: PCT/US2003/017751
(87) International publication number: WO 2004/000184

(56) References cited:
- WO-A-84/03833
- WO-A-89/07924
- DE-A- 3 815 506
- US-A- 4 081 884

## Description

### Cross-Reference to Related Applications

This invention is related to the following copending application:
US 2003/0233742 A1, filed June 25,2002, entitled"Compressed Absorbent Web".

### Field of the Invention

The present invention relates to a process for forming a densified structure using relatively low compression forces. The process includes heating an open structure prior to compression.

### Background of the Invention

Absorbent structures are manufactured under compression to provide sufficient absorbent capacity for a given use in a conveniently dimensioned product.
Absorbent structures may include wound care, diapers, sanitary napkins, tampons, and interlabial devices.

Many absorbent structures, such as tampons, achieve shape stability by slightly overcompressing the structure and allowing it to recover or expand to the desired dimensions. This structure may also be heat set. An example of this is described in Johst et al. , US Pat. No. 4,081, 884. This patent discloses radially compressing a tampon blank comprising cellulosic fibers, introducing the radially compressed tampon blank into a heated chamber, and axially compressing the tampon while heating for at least about five seconds. This process requires significant time to set the tampon.

Another example is disclosed in Wollangk et al., US Pat. No. 4,326, 527, which purports to disclose a process for rapidly and uniformly heat-setting a radially compressed tampon. The process includes the steps of compressing a prehumidified tampon and subjecting the compressed tampon to microwave heating while the tampon is in an open ended tube having openings about the longitudinal axis of the tube to heat-set the tampon. This process requires significant energy to set the tampon and the prehumidification may promote the growth of undesirable microorganisms during the manufacture of the tampon.

While the amount of compressive energy required in these references is not discussed in any great detail, it is our experience that the energy required to radially compress a commercial tampon, as measured by the compressive force, is very great.

This is especially true for non-conventional fiber blends, such as those containing non-cellulosic materials. This energy requirement can also limit the ability to commercially produce tampons having increased density. High compressive forces can damage process equipment and negatively affect the tampon performance by damaging the fibers within the tampon structure. This fiber damage can lead to poor expansion and absorbent capacity of the product.

In WO 84/03833 A1 a method for producing a hygiene product is disclosed which requires multiple fibers to be located in at least one outer layer of a non-woven material so that by applying heat to said outer layer the multiple fibers are fused together to form a net-like structure. Suitable multiple fibers comprised polyethylene, polypropylene, polyamide and polyester fibers. With the hygiene products obtained by the method of WO 84/03833 A1 it is reported to be possible to counter act the tendency of fibers to loosen there from.

In DE 38 15 506 A1 a process for preparing tampons from absorbent blanks which contain multiple fibers is disclosed. The multiple fibers are incorporated only into the rim portion of the blank so that upon fumation of the final tampon only the cover material comprises the multiple fibers. The method of DE 38 15 506 A1 requires only the rim portion of the tampon blank to be heated so that in the end not only there amount of heat multiple fibers can be reduced, but also energy needed for the melting process can be saved.

In WO 89/07924 A1 a method for manufacturing a tampon is described in which the tampon blank is produced by winding the filament or filaments of fiber material into a cross-play configuration on a mandrall which is caused to rotate relative to a filament guider which, in turn, is moved raisly procetingly relative to the mandrall in the longitudinal direction thereof. The mandrall is caused to rotate 0.1 to 5 revolutions during the time taken for the filament guider to move backwards and forward once. The obtained tampon blank is doffed from the mandrall prior to compression of the blank to its intended tampon form. The outer layers of the tampon blank of cross-play configuration can consist entirely of heat-sealable material which can be heated to heat-seeling temperature prior to compressing the tampon blank, preferably immediately prior to coiling the filament or filaments. In this manner the loosening of fibers is reported to be prevented.

Therefore, what is needed is a process for forming a compressed tampon that employs lower compressive force to reduce the risk of web and equipment damage.

### Summary of the Invention

It is an object of the present invention to provide process that can provide a compressed absorbent tampon web using lower compressive force than would otherwise be required to reduce the risk of web and equipment damage. This can produce a tampon having low density relaxation (as defined in the Examples).

It is another object of the present invention to provide a tampon having low density relaxation and that has a low degree of fiber damage despite being sufficiently compressed to form a tampon having an appropriate density.

In accordance with the present invention, there has been provided a novel process for forming a compressed tampon according to claim 1. The process includes the steps of forming an open structure comprising at least 5 wt-% of cellulosic materials; heating the open structure to a temperature in the range from 40°C to 100°C; compressing the heated open structure to form the compressed tampon; and releasing the compressed fibrous tampon from compression. Surprisingly, both the force required for compression and the degree of over-compression are significantly reduced when the fibrous web is heated prior to compression. Indeed, what we have found is that heating the fibrous web prior to compression into the tampon form provides a more consistent, dimensionally-controlled product. It also requires lower compression forces to achieve a dimensionally stable product with reduced fiber damage.

### Brief Description of the Drawing

Fig. 1 is a perspective view of a tampon of the present invention.
Fig. 2 is a diagrammatic view of an apparatus for producing a tampon according to one embodiment of the present invention.
Fig. 2A is a cross-section of carrier having a substantially cylindrical carrier for use in a modification of the apparatus of Fig. 2.
Fig. 3 is a diagrammatic view of an apparatus for producing a tampon according to another embodiment of the present invention.

### Detailed Description of the Preferred Embodiments

As used in the specification and claims, the term "open structure" and variations of this term relate to compressible structures prior to substantial compression to form compressed absorbent products, such as tampons. For example, these open structures may be formed by carding, air laying, or other processes and may include some minor calendering to maintain a density of less than about 0.1 g/cm³.

As used in the specification and claims, the term "compressible" and variations of this term relates to structures that can be compressed to hold a generally compressed form and that also can expand to a relatively uncompressed state upon exposure to sufficient moisture or liquid.

As used in the specification and claims, the term "radially expand" and variations of this term relate to the expansion of elongate tampons. These tampons expand primarily in a direction perpendicular to the central axis of the tampon. Preferably, the tampons expand in at least one direction perpendicular to the central axis, more preferably, at least two directions. Most preferably, the tampons expand substantially uniformly in all directions perpendicular to the central axis.

As used in the specification and claims, the term "axially expand" and variations of this term relates to the expansion of another particular class of elongate tampons. These tampons expand primarily in a direction along the central axis of the tampon. However, the tampons may also expand in at least one other direction.

The absorbent tampons of the present invention elongate masses of compressed materials, preferably substantially cylindrical masses of compressed materials having a central axis and a radius that defines the outer circumferential surface of the tampon. Tampons are often formed by first obtaining a shaped mass of materials called a tampon blank. This blank can be in the form of a roll of a nonwoven web, a mass of randomly or substantially uniformly oriented material.

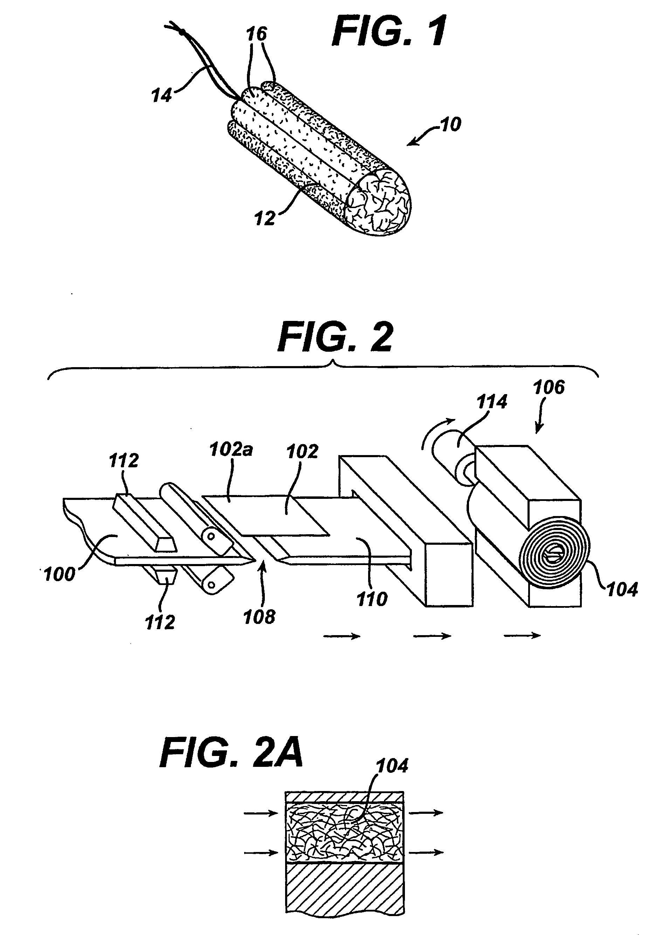

The tampon blank is an open structure that is relatively uncompressed and has a relatively low density. It is then compressed to form a product having smaller dimensions and a higher density than the tampon blank. After the tampon is released from compression, it relaxes (or expands), slightly, to its final dimensions. The compressed tampons may have a generally uniform density throughout the tampon, or they may have regions of differing density as described in the commonly assigned patents to Friese et al. , US Pat. No. 6,310, 269, and Leutwyler et al. , US Pat. No. 5, 911, 712. As shown in Fig.1, tampons 10 also usually include a cover 12 or some other surface treatment and a withdrawal string 14 or other removal mechanism.

The tampon 10 may have a relatively dense core substantially surrounding its central axis and a less dense annulus surrounding the core and forming the outer circumferential surface. This density differential may be provided by relatively uniform, yet distinct, absorbent material distribution within the core and annulus, or it may be provided by a plurality of ribs 16 which extend radially from the core.

The materials that may be used in the tampon include fibers, foams, and particles or other discrete materials. The tampon include cellulosic fibers. A useful, non-limiting list of useful cellulosic fibers includes natural fibers such as cotton, wood pulp, jute, hemp, sphagnum; and processed materials including cellulose derivatives such as regenerated cellulose (including rayon and lyocell), cellulose nitrate, carboxymethyl cellulose. The tampons may also include other materials including, without limitation, polyester, polyvinyl alcohol, polyolefin, polyamine, polyamide, polyacrylonitrile.

Preferably, the tampons are formed predominantly of fibers. The fibers may be any of the materials listed above, and may have any useful cross-section, including multi-limbed and non-limbed. Multi-limbed, regenerated cellulosic fibers have been commercially available for a number of years. These fibers are known to possess increased specific absorbency over non-limbed fibers. Commercial examples of these fibers are Danufil VY trilobal viscose rayon fibers available from Acordis Ltd., Spondon, England. These fibers are described in detail in Wilkes et al, US Pat. No. 5,458, 835.

The tampon includes at least about 5 wt-% of the cellulosic materials. These materials are moisture sensitive, and provide hydrogen bonding when compressed under moist conditions. More preferably, the tampon includes about 35 to about 100 wt-% cellulosic materials, and most preferably, about 50 to about 75 wt-% cellulosic materials.

Prior to heating, the open structure has a moisture content of at least about 4 wt-%, preferably, about 8 to about 13 wt-%. After heating, the open structure retains sufficient moisture content to promote interfiber bonds sufficient to maintain the dimensions of the compressed tampon. The open structure has a moisture content of about 2 to about 13 wt-% after heating.

Preferably, the tampon blank is substantially enclosed by a fluid-permeable cover. Thus, the cover encloses a majority of the outer surface of the tampon. This may be achieved as disclosed in Friese, U. S. Patent No. 4,816, 100. In addition, either or both ends of the tampon may be enclosed by the cover. Of course, for processing or other reasons, some portions of the surface of the tampon may be free of the cover. For example, the insertion end of the tampon and a portion of the cylindrical surface adjacent this end may be exposed, without the cover to allow the tampon to more readily accept fluids.

The cover can ease the insertion of the tampon into the body cavity and can reduce the possibility of fibers being separated from the tampon. Those of ordinary skill in the art will recognize covers that are useful in conjunction with the tampons of the present invention. They may be selected from an outer layer of fibers which are fused together (such as bythermobonding), a nonwoven fabric, an apertured film, or the like.

Tampons are generally categorized in two classes: applicator tampons and digital tampons, and a certain amount of dimensional stability is useful for each type of tampon. Applicator tampons use a relatively rigid device to contain and protect the tampon prior to use. To insert the tampon into a body cavity, the applicator is partially inserted into the body cavity, and the tampon can be expelled therefrom. In contrast, digital tampons do not have an applicator to help guide them into the body cavity and require sufficient column strength to allow insertion without using an applicator. This strength can be determined by securing one end of the tampon to the fixed plate of a Instron Universal Testing Machine, available from Instron Corporation, Canton, Massachusetts, USA. The moveable plate is brought to contact the opposite end of the tampon and is then set to compress the tampon at a rate of about 5cm/minute. The force exerted on the tampon is measured continuously, and the point at which this force begins to fall instead of rise is the point at which the tampon buckles. The maximum force achieved is the tampon stability. Preferably, digital tampons of the present invention have a significant stability, at least about 10 N. More preferably, the digital tampons have a stability of at least about 20 N, and most preferably, they have a stability of about 30 N to about 85 N. Tampons with a stability that is too low do not have sufficient dimensional stability to maintain their basic structure during insertion as a digital tampon; tampons with a stability which is too high can be perceived as being too stiff or hard to be comfortably inserted as a digital tampon.

While the applicator tampon is protected by the rigid applicator device and the applicator tampon need not as have high a degree of column strength as a digital tampon, applicator tampons do require dimensional stability (especially radial) to be acceptable for use. This dimensional stability provides assurance, for example, that the tampon will not prematurely grow and split its packaging material or become wedged in a tampon applicator.

The process of the present invention begins with an open structure. The open structure is a nonwoven web, a mass of randomly or substantially uniformly oriented materials, such as fibers, foams, or particles. This mass is then manipulated to form a tampon blank.

A nonwoven web useful in the present invention can be formed in any manner desired by the person of ordinary skill in the art. For example, fibers can be openedand/or blended by continuously metering them into a saw-tooth opener. The blended fibers can be transported, e. g. , by air through a conduit to a carding station to form a fibrous web. Alternatively, a mass of substantially randomly oriented fibers can be formed by openingand/or blending them, transporting them, as above, to a station to form, e. g. , a teabag-type tampon blank. Further processes may employ oriented fibers in a fibrous tow.

The tampon blank can be further processed to form a tampon. In a tampon forming process, a web can be formed into a narrow, fibrous sliver and spirally wound to form a tampon blank. In addition, a liquid-permeable cover material can be wrapped around the tampon blank to substantially contain the fibrous absorbent portion of the tampon. Examples of the further processing of the webs are described in Friese et al., US Pat. No. 4,816, 100, and Schwankhardt, US Pat. No. 5,909, 884. However, these processes are to be modified according to the present invention.

The open structure may be heated prior to its formation into a tampon blank. The open structure may also be heated after its formation into a tampon blank, or even both before and after. The resulting pre-heated tampon blank can then be compressed at a significantly reduced pressure to form a dimensionally stable tampon.hi the process of Friese et al. , a fibrous web 100, having a width corresponding to the length of the tampon 10, is supplied continuously and is weakened transversely to its longitudinal direction. This weakening may be achieved through perforating and stretching of the web to reduce its cross-section at a weakened zone. A continuously supplied cover strip is severed to form a cover section 102, the length of which exceeds the circumference of the tampon blank 104 as shown in the winding station 106. The cover section 102 is bonded (e. g., thermally) sealed to the outside of a region of the web 100 at one end of the web section adjacent the weakened zone. The cover section 102 is arranged on the web 100 such that a free end 102a of the cover section 102 extends beyond the weakened zone. The web 100 can then be severed at the weakened zone to form a gap 108 between adjacent web sections 110.

The web 100 can be heated by means of heaters 112, which may, e. g. , be placed prior to the severing of adjacent web sections 110. A heated web sections 110 can then be wound upon itself about an axis extending transversely to its longitudinal direction by a winding mandrel 114. This forms a tampon blank 104. The wound-up tampon blank 104 can efficiently retain the applied heat due to an insulating effect of the outer layers of the web. This may be enhanced by enclosing the process equipment around the heated tampon blank and/or heated web sections.

Alternatively, heated air can be forced through the relatively loosely wound-up tampon blank 104 to preheat the blank prior to compression as shown in Fig. 2A.

Heat can be applied to the fibrous mass or web via conduction, convection, radiation, combinations of these. Such processes include, without limitation, circulation of hot air or steam, electromagnetic transmission of energy (for example, without limitation, radio frequency energy, infrared energy, microwave energy), insertion of heated pins into web to provide conductive heat transfer, ultrasonic energy, and the like. The open structure is heated to a temperature of at least about 40°C. More preferably, the open structure is heated to at least about 45°C, and most preferably, the open structure is heated to at least about 60°C. In order to avoid overheating some thermoplastic fibers or over-drying the structure, the temperature of the open structure is limited to less than about 100°C or even 85°C.

After winding, the cover section 102 completely surrounds the circumference of the tampon blank 104 over the intended width, and the free end 102a can be thermally bonded to an overlapped portion of itself on the outside of the tampon blank 104.

A withdrawal string can be placed around the web section 110 prior to winding and, if appropriate, knotted at its free ends. The finished tampon blank 104 can then be delivered to a tampon press, as is disclosed in Leutwyler et al. , US Pat. No. 5,911, 712.

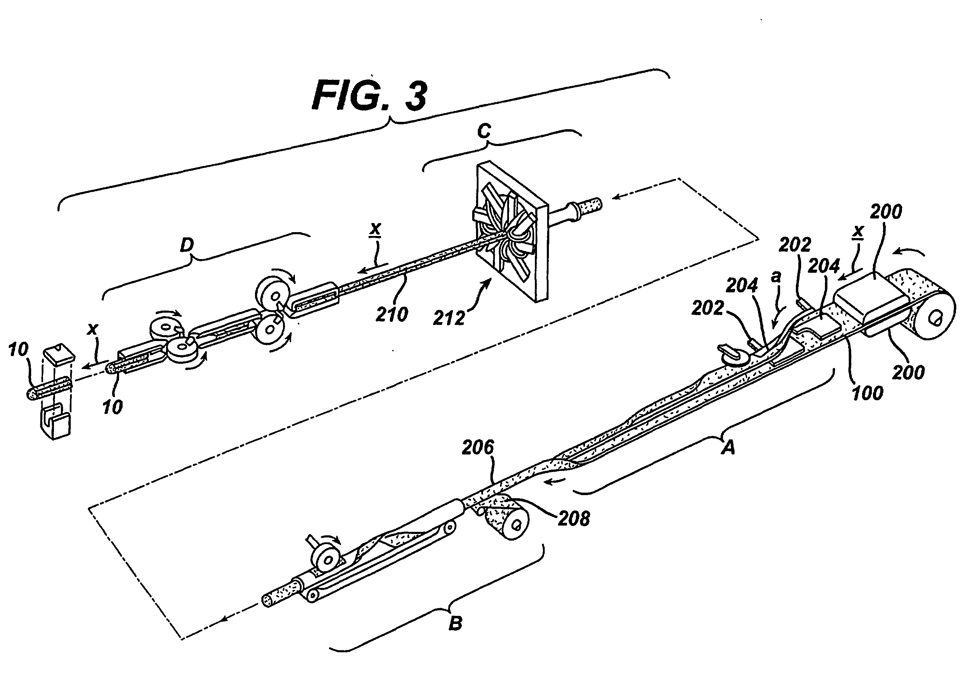

As shown in Fig. 3, coincident with the process of Schwankhardt, a fibrous web 100 can be heated by a heater 200 prior to entering the folding station A in which a series of folding plates 202 and baffle plates 204 sequentially fold the web to form a folded or essentially wound-up fibrous rope 206 as it exits the folding station. The fibrous rope 206 can be enveloped in a cover material 208 (a wrapping band according toSchwankhardt) in a wrapping station B, compressed into a compressed strand 210 in a press 212 of a press station C, and cut andformed into individual tampons 10 in a severing station D, and packaged. The heating is preferably performed prior to the first folding plate 202 and baffle plate 204 to allow for substantially uniform heating through the thickness of the web 100. However, the heating can be performed or supplemented further into the folding station, preferably before too many layers of the web are folded up.

In addition to the processes described above, the processes of manufacturing tampons disclosed in Haas, US Pat. No. 1,926, 900, Voss, US Pat. No. 2,076,389, and Dostal, US Pat. No. 3,811, 445, can be modified in similar fashions to that described above to take advantage of the inventive concepts herein disclosed.

As described above, tampons are generally over-compressed to mechanically constrict spontaneous expansion of the structure thereby preventing the tampon from expanding too much before use. However, this over-compression is not always uniform, and its effectiveness varies. In particular, when large masses of fibers are compressed, localized volumes may be subjected to greater compressive forces than other volumes. This may be desirable as in the Friese and Leutwyler disclosures, described above. Unfortunately, it can also result in compression being concentrated in the outer regions of a tampon and result in less control of the dimensional stability of the tampon.

Surprisingly, both the force required for compression and degree of over-compression are significantly reduced when the fibrous web is heated prior to compression. This is also reflected in the lower density relaxation. Based upon these findings, we expect that heating the fibrous web prior to compression into the tampon form provides a more consistent, dimensionally-controlled product. It also requires lower compression forces to achieve a dimensionally stable product.

One way to illustrate the consistency and dimensional control of the tampon is a review of the density relaxation (as defined below in the Examples) of the tampon. Preferably, the tampons of the present invention have a density relaxation of less than about 20%, more preferably, less than about 10%, and most preferably, less than about 5%.

Another way to illustrate the advantages of the present invention is the reduced fiber damage in the tampon. Fiber damage, including permanent fiber deformation and breakage, occurs during compression of the tampon. Fiber damage can be determined by examining the tampon for fibers that have been broken. For example, tampons that are formed of staple length fibers (about 1 to 1.5 inches (25 to about 40 mm)) can be inspected to determine the number or percentage of fibers that have a length of less than about ¾ inch (18 mm). Alternatively, these tampons can be analyzed to determine the percentage of fines (fibers having a length of less than about ¼ inch (7 mm)). A significant percentage of short fibers or fines can be indicative of fiber damage in a product.

After heating, the open structure and/or tampon blank beneficially retains its heat due to the inherent insulating properties of a loosely gathered mass of fibers and the heated air trapped in the capillaries thereof. Looser capillaries of the more open web allow more even heat conduction into center of the web. Further, when the unrolled web is heated there is less thermal mass between the heat source and the center of the web than there is when the heat is provided to the compressed tampon. Of course, the applied heat can dissipate into the atmosphere if compression does not follow the heating within a reasonable time.

While we have found that the above process can provide sufficient dimensional stability without additional post-compression heating, some amount of post-compression heating may be desirable to optimize a manufacturing process.

However, any such post-compression heat required is greatly reduced over prior art processes which do not employ any pre-compression heating.

We also believe that the present process allows for increased manufacturing line speeds and improved processability. For example, we have found that early fiber heating decreases the amount of materials, such as fibers, lost from the open structure during pre-compression handling. This produces a more consistent material stream leading into later processing stations.

### Examples

The present invention will be further understood by reference to the following specific Examples that are illustrative of the composition, form and method of producing the device of the present invention. It is to be understood that many variations of composition, form and method of producing the device would be apparent to those skilled in the art. The following Examples, wherein parts and percentages are by weight unless otherwise indicated, are only illustrative.

### Example 1

A blend of 75 wt-% 1/3tex Danufil®; VY trilobal viscose rayon fibers and 25 wt-% 1/3 tex Danufil® V viscose rayon fibers, both available from Acordis Ltd., Spondon, England, were opened via standard fiber opening and carding equipment. A fixed amount of the fiber blend (having a mass, W, of about 2 g) was introduced in a stainless steel mold with a cylindrical cavity (of cross-sectional area, A, of about 5 cm²). A cylindrical plunger size matched to the cylindrical cavity was used to compress the fiber mass using a standard laboratory press. In order to heat the samples, the mold and plunger were heated together in an oven set at the target temperature. After sufficient time to allow the mold and plunger to reach the oven temperature, the fibers were placed into the cavity, and the mold, plunger and fibers were heated for an additional three minutes to allow the fibers to reach the oven temperature. The heated assembly was removed from the oven and placed between the plates of the laboratory press. Pressure was applied to compress the fiber mass in the cavity up to a predetermined peak pressure and released, after which the compressed fibrous plug was removed to allow an immediate measurement of the initial thickness, To.

The compressed fibrous plug had an initial volume (V₀ = A * T₀) and an initial density (ρ₀ = W/V₀), but the plug expanded after the pressure was released, reaching equilibrium after about 15 to 20 minutes (at room temperature, approximately 20°C). While the humidity conditions of the test are not generally critical, conducting the test at high humidity will adversely affect the test results. The equilibrium thickness, Tₑ, was then measured to provide an equilibrium density (ρₑ = W/(A * Tₑ)). From these values, a "Density relaxation" can be calculated equal to (ρ₀ - ρₑ)/ ρ₀.

A control was also prepared employing mold, plunger and fibers at room temperature, about 20°C. The results of measurements at each temperature and pressure are shown in Table 1.

**TABLE 1**

| Temperature | Peak Pressure | Initial Density | Equilibrium Density | % Density relaxation |
|---|---|---|---|---|
| 100°C | 610 | 0.46 | 0.45 | 2% |
| | 1200 | 0.62 | 0.62 | < 2% |
| | 1800 | 0.75 | 0.75 | < 2% |
| | 2500 | 0.80 | 0.80 | < 2% |
| | 3000 | 0.85 | 0.84 | < 2% |
| | 3600 | 0.88 | 0.88 | < 2% |
| | 4800 | 0.92 | 0.92 | < 2% |
| | 6100 | 0.95 | 0.95 | < 2% |
| | | | | |
| 85°C | 610 | 0.34 | 0.34 | < 2% |
| | 910 | 0.49 | 0.49 | < 2% |
| | 1200 | 0.43 | 0.43 | < 2% |
| | 1500 | 0.55 | 0.55 | < 2% |
| | 1800 | 0.53 | 0.53 | < 2% |
| | 3600 | 0.85 | 0.86 | < 2% |
| | 4900 | 0.85 | 0.86 | < 2% |
| | | | | |
| 75°C | 610 | 0.36 | 0.36 | < 2% |
| | 910 | 0.40 | 0.39 | 3% |
| | 1200 | 0.52 | 0.51 | 2% |
| | 1500 | 0.54 | 0.53 | < 2% |
| | 1800 | 0.65 | 0.64 | < 2% |
| | 3600 | 0.80 | 0.80 | < 2% |
| | 4900 | 0.84 | 0.84 | < 2% |
| | 6100 | 0.91 | 0.92 | < 2% |
| | | | | |
| 60°C | 910 | 0.33 | 0.32 | 3% |
| | 1200 | 0.41 | 0.40 | 2% |
| | 1500 | 0.44 | 0.44 | < 2% |
| | 1800 | 0.49 | 0.47 | 4% |
| | 2200 | 0.57 | 0.55 | 4% |
| | 3000 | 0.65 | 0.64 | < 2% |
| | 4900 | 0.79 | 0.78 | < 2% |
| | 6100 | 0.87 | 0.87 | < 2% |
| | | | | |
| 40°C | 1200 | 0.31 | 0.30 | 3% |
| | 1800 | 0.43 | 0.40 | 7% |
| | 2400 | 0.50 | 0.47 | 6% |
| | 3000 | 0.58 | 0.56 | 3% |
| | 4300 | 0.69 | 0.66 | 4% |
| | | | | |
| ROOM | 1200 | 0.23 | 0.17 | 26% |
| | 2400 | 0.33 | 0.25 | 24% |
| | 3600 | 0.50 | 0.38 | 24% |
| | 4900 | 0.60 | 0.46 | 23% |
| | 6200 | 0.64 | 0.51 | 20% |

These data show that pre-heating of fibers at a temperature of at least about40 C and maintaining the heat during compression provides a significantly greater dimensional stability than compressing the same fibers at room temperature. They further illustrate that substantially higher fiber plug densities can be achieved at lower compression pressures when fibers are pre-heated. This is even more pronounced at temperatures of greater than about 60°C.

### Example 2

The procedure of Example 1 was repeated with a blend of 75 wt-% 1/3 tex Danufil® V viscose rayon fibers, available from Acordis Ltd. (Spondon, England), and 25wt-% 1/3 tex T-224 polyester fibers, available from RoSa, (Houston, Texas, USA). Again, the results of measurements at each temperature and pressure are shown in Table 2.

**TABLE 2**

| Temperature | Peak Pressure | Initial Density | Equilibrium Density | % Density relaxation |
|---|---|---|---|---|
| 100°C | 610 | 0.33 | 0.34 | <2% |
| | 1200 | 0.47 | 0.48 | <2% |
| | 7400 | 0.62 | 0.63 | < 2% |
| | 3000 | 0.65 | 0.65 | < 2% |
| | | | | |
| 85°C | 910 | 0.36 | 0.35 | 3% |
| | 1200 | 0.39 | 0.39 | < 2% |
| | 2400 | 0.53 | 0.53 | < 2% |
| | 3600 | 0.66 | 0.66 | < 2% |
| | 4900 | 0.80 | 0.79 | <2% |
| | 6100 | 0.79 | 0.78 | < 2% |
| | | | | |
| 75°C | 910 | 0.33 | 0.32 | 3% |
| | 1200 | 0.37 | 0.37 | < 2% |
| | 2400 | 0.52 | 0.50 | 4% |
| | 3600 | 0.65 | 0.64 | < 2% |
| | 4900 | 0.71 | 0.70 | < 2% |
| | 6100 | 0.79 | 0.80 | < 2% |
| | | | | |
| 60°C | 610 | 0.25 | 0.24 | 4% |
| | 1200 | 0.38 | 0.37 | 3% |
| | 2400 | 0.50 | 0.50 | < 2% |
| | 3600 | 0.62 | 0.60 | 3% |
| | 6100 | 0.77 | 0.75 | 3% |
| | | | | |
| 45°C | 910 | 0.30 | 0.29 | 3% |
| | 1200 | 0.34 | 0.34 | < 2% |
| | 2400 | 0.44 | 0.43 | 2% |
| | 3600 | 0.59 | 0.59 | < 2% |
| | 4900 | 0.71 | 0.69 | 3% |
| | 6100 | 0.77 | 0.76 | < 2% |
| | | | | |
| ROOM | 2400 | 0.41 | 0.33 | 20% |
| | 3600 | 0.51 | 0.40 | 22% |
| | 3800 | 0.55 | 0.40 | 27% |
| | 3800 | 0.52 | 0.40 | 23% |
| | 4900 | 0.61 | 0.52 | 15% |

These data show that pre-heating of fibers at a temperature of at least about 45°C and maintaining the heat during compression provides a significantly greater dimensional stability than compressing the same fibers at room temperature. They further illustrate that substantially higher fiber plug densities can be achieved at lower compression pressures when fibers are pre-heated. This is even more pronounced at temperatures of greater than about 60°C. However, with thermoplastic fibers, such as polyester fibers, this preheating may be limited to avoid exceeding their yield point to cause permanent deformation of the fibers, including melting the fibers.

### Example 3

The procedure of Example 1 was repeated with different blends of 1/3 tex Danufil® V viscose rayon fibers, available from Acordis Ltd. (Spondon, England), and 3 tex T-224 polyester fibers, available from KoSa, (Houston, Texas, USA).
However, in this series, the temperature was maintained at 75°C, while the proportion of fibers varied. The results of measurements at each blend and pressure are shown in Table 3.

**TABLE 3**

| | Peak Pressure | Initial Density | Equilibrium Density | % Density relaxation |
|---|---|---|---|---|
| 25% PET | 910 | 0.33 | 0.32 | 3% |
| | 1200 | 0.37 | 0.37 | < 2% |
| | 2400 | 0.52 | 0.50 | 4% |
| | 3600 | 0.65 | 0.64 | <2% |
| | 4900 | 0.71 | 0.70 | <2% |
| | 6100 | 0.79 | 0.80 | <2% |
| | | | | |
| 33% PET | 610 | 0.24 | 0.23 | 4% |
| | 910 | 0.31 | 0.30 | 3% |
| | 1200 | 0.39 | 0.38 | 3% |
| | 2400 | 0.49 | 0.47 | 4% |
| | | | | |
| 50% PET | 910 | 0.28 | 0.28 | <2% |
| | 1200 | 0.32 | 0.31 | 3% |
| | 2400 | 0.49 | 0.47 | 4% |
| | 3600 | 0.59 | 0.57 | 3% |
| | 4900 | 0.68 | 0.67 | < 2% |
| | 6100 | 0.75 | 0.75 | < 2% |
| | | | | |
| 67% PET | 1200 | 0.31 | 0.30 | 3% |
| | 2400 | 0.41 | 0.41 | < 2% |
| | 3600 | 0.63 | 0.62 | < 2% |
| | 6100 | 0.70 | 0.69 | <2% |

These data show that pre-heating of fibers at a temperature of about 75°C and maintaining the heat during compression provides significant dimensional stability, even with large proportions of relatively resilient fibers, such as polyester.

The specification and embodiments above are presented to aid in the complete and non-limiting understanding of the invention disclosed herein. Since many variations and embodiments of the invention can be made without departing from its scope, the invention resides in the claims hereinafter appended.

## Claims

1. A process for forming a compressed tampon comprising the steps:
- forming an open structure in the form of a non-woven web or a mass of randomly or substantially uniformly oriented materials representing a compressible structure prior to substantial compression to form a tampon as a compressed absorbent product, said open structure having a density of less than 0,1 g/cm³, and a moisture content of at least 4 wt.-% prior to heating, and comprising at least 5 wt-% of cellulosic materials;
- forming a tampon blank from the non-woven web or the mass of randomly or substantially uniformly oriented materials;
- wherein said non-woven web or said mass of randomly or substantially uniformly oriented materials and/or said tampon blank is heated resulting in a heated open structure having a temperature in the range from 40°C to 100°C when subjected to compression,
while substantially maintaining moisture present in the open structure, wherein the open structure has a moisture content of 2 to 13 wt.-% after heating;
- compressing said open structure to form the compressed tampon; and
- releasing the compressed tampon from compression.

2. The process of claim 1 wherein the open structure comprises at least 15 wt-% of cellulosic materials.

3. The process of claim 1 wherein the open structure comprises from 50 to 75 wt-% of cellulosic materials.

4. The process of claim 1 wherein the open structure further comprises at least 5 wt-% non-cellulosic polymeric materials.

5. The process of claim 1 comprising heating the open structure to a temperature of at least 45° C.

6. The process of claim 5 comprising heating the open structure to a temperature of at least 60° C.

7. The process of claim 1, wherein the open structure is heated to a temperature less than 85°C.

8. The process of claim 1, wherein prior to heating the open structure has a moisture content of 8 to 13 wt-%.

9. The process of claim 1 which further comprises the step of forming the open structure into an elongate shape.

10. The process of claim 9 wherein the open structure is heated prior to forming the open structure into an elongate shape.

11. The process of claim 9 wherein the open structure is formed into an elongate shape prior to heating.

12. The process of claim 9 wherein the open structure is a fibrous web, and the step of forming the open structure into an elongate shape comprises winding the fibrous web to form a substantially cylindrical shape.

13. The process of claim 1 comprising the step of compressing the heated open structure to form the compressed tampon to a density of greater than 0.3 g/cm³.

14. The process of claim 13 comprising the step of compressing the heated open structure to form the compressed tampon to a density of greater than 0.4g/cm³.

15. The process of claim 1 wherein the cellulosic materials comprise cellulosic fibers.

16. The process of claim 4 wherein the non-cellulosic polymeric materials comprise fibers.

17. The process of claim 1 wherein the step of heating the open structure comprises moving heated air through the open structure.

18. The process of claim 17 wherein the heated air is humidified.

19. The process of claim 1 wherein the step of heating the open structure comprises applying electromagnetic energy to the open structure.

20. The process of claim 19 wherein the electromagnetic energy is selected from the group consisting of infrared radiation, ultrasonic energy, and microwave energy.

21. The process of claim 1 further comprising the step of enclosing the compressed tampon in packaging material.

22. The process of claim 1 wherein the compressed tampon has a density relaxation of less than 20 %, wherein the density relaxation is calculated to be equal to (pₒ- pₑ)/pₒ, the initial density is calculated equal to (pₒ = W/Vₒ), the initial volume is calculated to be equal to (Vₒ = A * Tₒ), and Tₒ is the initial thickness of said tampon, and wherein the equilibrium density is calculated equal to (pₑ = W/Vₑ), the equilibrium volume is calculated to be equal to (Vₑ = A * Tₑ) and Tₑ is the equilibrium thickness of the tampon.

23. The process of claim 22 wherein the compressed tampon has a density relaxation of less than 10 %.

24. The process of claim 22 wherein the compressed tampon has a density relaxation of less than 5%.

## Patentansprüche

1. Verfahren für die Herstellung eines komprimierten Tampons, welches die folgenden Schritte umfasst:
- Ausbildung einer offenen Struktur in Form eines Vliesgewebes oder einer Masse von willkürlich oder im wesentlichen einheitlich ausgerichteten Materialien, welche eine komprimierbare Struktur vor der eigentlichen Komprimierung zur Ausbildung eines Tampons als komprimiertes absorbierendes Produkt darstellen, wobei die offene Struktur eine Dichte von weniger als 0,1 g/cm3 und einen Feuchtigkeitsgehalt von zumindest 4 Gewichtsprozent vor dem Erwärmen aufweist und zumindest 5 Gewichtsprozent Zellulosematerialien umfasst;
- Ausbildung eines Tamponrohlings aus dem Vliesgewebe oder der Masse von willkürlich oder im wesentlichen einheitlich ausgerichteten Materialien;
- wobei das Vliesgewebe oder die Masse von willkürlich oder im wesentlichen einheitlich ausgerichteten Materialien und/oder der Tamponrohling erwärmt wird und zu einer erwärmten offenen Struktur mit einer Temperatur im Bereich von 40° C bis 100° C wird , wenn sie der Komprimierung ausgesetzt wird, während die in der offenen Struktur vorliegende Feuchtigkeit im wesentlichen beibehalten wird,
wobei die offene Struktur nach der Erwärmung einen Feuchtigkeitsgehalt von 2 bis 13 Gewichtsprozent aufweist;
- Komprimierung der offenen Struktur zur Ausbildung des komprimierten Tampons; und
- Freigabe des komprimierten Tampons aus der Komprimierung.

2. Verfahren nach Anspruch 1, **dadurch gekennzeichnet, dass** die offene Struktur zumindest 15 Gewichtsprozent Zellulosematerialien umfasst.

3. Verfahren nach Anspruch 1, **dadurch gekennzeichnet, dass** die offene Struktur von 50 bis 75 Gewichtsprozent Zellulosematerialien umfasst.

4. Verfahren nach Anspruch 1, **dadurch gekennzeichnet, dass** die offene Struktur weiterhin zumindest 5 Gewichtsprozent an nicht-Zellulose-Polymermaterialien umfasst.

5. Verfahren nach Anspruch 1, welches die Erwärmung der offenen Struktur auf eine Temperatur von zumindest 45° C umfasst.

6. Verfahren nach Anspruch 5, welches die Erwärmung der offenen Struktur auf eine Temperatur von zumindest 60° C umfasst.

7. Verfahren nach Anspruch 1, **dadurch gekennzeichnet, dass** die offene Struktur auf eine Temperatur von weniger als 85° C erwärmt wird.

8. Verfahren nach Anspruch 1, **dadurch gekennzeichnet, dass** vor dem Erwärmen die offene Struktur einen Feuchtigkeitsgehalt von 8 bis 13 Gewichtsprozent aufweist.

9. Verfahren nach Anspruch 1, welches weiterhin den Schritt der Ausbildung der offenen Struktur zu einer länglichen Form umfasst.

10. Verfahren nach Anspruch 9, **dadurch gekennzeichnet, dass** die offene Struktur vor der Ausbildung der offenen Struktur zu einer länglichen Form erwärmt wird.

11. Verfahren nach Anspruch 9, **dadurch gekennzeichnet, dass** die offene Struktur vor dem Erwärmen zu einer länglichen Form ausgebildet wird.

12. Verfahren nach Anspruch 9, **dadurch gekennzeichnet, dass** die offene Struktur ein faseriges Gewebe ist und dass der Schritt der Ausbildung der offenen Struktur zu einer länglichen Form das Aufwickeln des faserigen Gewebes zu einer im wesentlichen zylindrischen Form umfasst.

13. Verfahren nach Anspruch 1, welches den Schritt der Komprimierung der erwärmten offenen Struktur umfasst, um den komprimierten Tampon mit einer Dichte von mehr als 0,3 g/cm³ auszubilden.

14. Verfahren nach Anspruch 13, welches den Schritt der Komprimierung der erwärmten offenen Struktur umfasst, um den komprimierten Tampon mit einer Dichte von mehr als 0,4 g/cm³ auszubilden.

15. Verfahren nach Anspruch 1, **dadurch gekennzeichnet, dass** die Zellulosematerialien Zellulosefasern umfassen.

16. Verfahren nach Anspruch 4, **dadurch gekennzeichnet, dass** die nicht-Zellulose-Polymermaterialien Fasern umfassen.

17. Verfahren nach Anspruch 1, **dadurch gekennzeichnet, dass** der Schritt der Erwärmung der offenen Struktur das Durchleiten erwärmter Luft durch die offene Struktur umfasst.

18. Verfahren nach Anspruch 17, **dadurch gekennzeichnet, dass** die erwärmte Luft angefeuchtet ist.

19. Verfahren nach Anspruch 1, **dadurch gekennzeichnet, dass** der Schritt des Erwärmens der offenen Struktur das Aufbringen elektromagnetischer Energie auf die offene Struktur umfasst.

20. Verfahren nach Anspruch 19, **dadurch gekennzeichnet, dass** die elektromagnetische Energie ausgewählt wird aus der Gruppe, bestehend aus Infrarotstrahlung, Ultraschallenergie und Mikrowellenenergie.

21. Verfahren nach Anspruch 1, welches weiterhin den Schritt der Umhüllung des komprimierten Tampons mit Verpackungsmaterial umfasst.

22. Verfahren nach Anspruch 1, **dadurch gekennzeichnet, dass** der komprimierte Tampon eine Dichterelaxation von weniger als 20 % aufweist, wobei die Dichterelaxation so berechnet ist, dass sie (pₒ-p_{c})/Pₒ entspricht, wobei die anfängliche Dichte gemäß (pₒ=W/Vₒ) berechnet wird, das anfängliche Volumen gemäß (Vₒ=A*Tₒ) berechnet wird und Tₒ die anfängliche Dicke des Tampons ist und
wobei die Gleichgewichtsdichte gemäß (pₑ=W/Vₑ) berechnet wird, das Gleichgewichtsvolumen gemäß (Vₑ=A*Tₑ) berechnet wird und Tₑ die Gleichgewichtsdicke des Tampons ist.

23. Verfahren nach Anspruch 22, **dadurch gekennzeichnet, dass** der komprimierte Tampon eine Dichterelaxation von weniger als 10 % aufweist.

24. Verfahren nach Anspruch 22, **dadurch gekennzeichnet, dass** der komprimierte Tampon eine Dichterelaxation von weniger als 5 % aufweist.

## Revendications

1. Processus de formation d'un tampon comprimé, comprenant les étapes consistant à :
■ former une structure ouverte sous la forme d'une bande non tissée ou d'une masse de matériaux orientés de manière aléatoire ou substantiellement uniforme représentant une structure compressible avant la compression substantielle pour former un tampon en tant qu'un produit absorbant comprimé, ladite structure ouverte ayant une densité inférieure à 0,1 g/cm³, et une teneur en eau d'au moins 4 % en poids avant le chauffage, et comprenant au moins 5 % en poids de matériaux cellulosiques ;
■ former une ébauche de tampon à partir de la bande non tissée ou de la masse de matériaux orientés de manière aléatoire ou substantiellement uniforme ;
dans lequel ladite bande non tissée ou ladite masse de matériaux orientés de manière aléatoire ou substantiellement non uniforme et/ou ladite ébauche de tampon sont chauffées résultant en une structure ouverte chaude ayant une température dans la plage de 40°C à 100°C lorsqu'elle est soumise à une compression,
tout en maintenant substantiellement la présence d'eau dans la structure ouverte, dans lequel la structure ouverte a une teneur en eau de 2 à 13 % en poids après le chauffage ;
■ comprimer ladite structure ouverte pour former le tampon comprimé ; et
■ libérer le tampon comprimé de la compression.

2. Processus selon la revendication 1, dans lequel la structure ouverte comprend au moins 15 % eh poids de matériaux cellulosiques.

3. Processus selon la revendication 1, dans lequel la structure ouverte comprend de 50 à 75 % en poids de matériaux cellulosiques.

4. Processus selon la revendication 1, dans lequel la structure ouverte comprend en outre au moins 5 % en poids de matériaux polymères non cellulosiques.

5. Processus selon la revendication 1, comprenant le chauffage de la structure ouverte à une température d'au moins 45°C.

6. Processus selon la revendication 5, comprenant le chauffage de la structure ouverte à une température d'au moins 60°C.

7. Processus selon la revendication 1, dans lequel la structure ouverte est chauffée à une température inférieure à 85°C.

8. Processus selon la revendication 1, dans lequel avant le chauffage, la structure ouverte a une teneur en eau de 8 à 13 % en poids.

9. Processus selon la revendication 1, qui comprend en outre l'étape consistant à mettre la structure ouverte sous une forme allongée.

10. Processus selon la revendication 9, dans lequel la structure ouverte est chauffée avant d'être mise sous une forme allongée.

11. Processus selon la revendication 9, dans lequel la structure ouverte est mise sous une forme allongée avant d'être chauffée.

12. Processus selon la revendication 9, dans lequel la structure ouverte est une bande fibreuse, et l'étape consistant à mettre la structure ouverte sous une forme allongée comprend l'enroulement de la bande fibreuse pour former une forme substantiellement cylindrique.

13. Processus selon la revendication 1, comprenant l'étape consistant à comprimer la structure ouverte chaude pour former le tampon comprimé à une densité supérieure à 0,3 g/cm³.

14. Processus selon la revendication 13, comprenant l'étape consistant à comprimer la structure ouverte chaude pour former le tampon comprimé à une densité supérieure à 0,4 g/cm³.

15. Processus selon la revendication 1, dans lequel les matériaux cellulosiques comprennent des fibres cellulosiques.

16. Processus selon la revendication 4, dans lequel les matériaux polymères non cellulosiques comprennent des fibres.

17. Processus selon la revendication 1, dans lequel l'étape consistant à chauffer la structure ouverte comprend le déplacement d'air chaud dans la structure ouverte.

18. Processus selon la revendication 17, dans lequel l'air chaud est humidifié.

19. Processus selon la revendication 1, dans lequel l'étape consistant à chauffer la structure ouverte comprend l'application d'énergie électromagnétique à la structure ouverte.

20. Processus selon la revendication 19, dans lequel l'énergie électromagnétique est choisie dans le groupe constitué par les rayonnements infrarouges, l'énergie ultrasonore, et l'énergie micro-onde.

21. Processus selon la revendication 1, comprenant en outre l'étape consistant à enfermer le tampon comprimé dans un matériau d'emballage.

22. Processus selon la revendication 1, dans lequel le tampon comprimé a une relaxation de la densité inférieure à 20 %, dans lequel la relaxation de la densité est calculée comme étant égale à (ρₒ-ρₑ) / ρₒ; la densité initiale est calculée égale à (ρ = W/Vₒ), le volume initial est calculé comme étant égal à (Vₒ = A * Tₒ), et Tₒ est l'épaisseur initiale dudit tampon, et dans lequel la densité d'équilibre est calculée égale à (ρₑ = W/Vₑ), le volume d'équilibre est calculé comme étant égal à (Vₑ = A * Tₑ) et Tₑ est l'épaisseur d'équilibre du tampon.

23. Processus selon la revendication 22, dans lequel le tampon comprimé a une relaxation de la densité inférieure à 10 %.

24. Processus selon la revendication 22, dans lequel le tampon comprimé a une relaxation de la densité inférieure à 5 %.
